# EUROPÄISCHE PATENTSCHRIFT

(11) **EP 1 008 586 B1**
(45) Veröffentlichungstag und Bekanntmachung des Hinweises auf die Patenterteilung: **06.08.2003**
(21) Anmeldenummer: 99122878.4
(22) Anmeldetag: 18.11.1999
(51) Int. Cl.: C07C 255/41, C07C 69/618, A61K 7/42

(54) **Oligomere Diarylbutadiene**
Oligomeric diarylbutadienes
Diarylbutadiènes oligomères

(30) Priorität: 11.12.1998 DE 19857127
(43) Veröffentlichungstag der Anmeldung: 14.06.2000
(73) Patentinhaber: BASF AKTIENGESELLSCHAFT, 67056 Ludwigshafen (DE)
(72) Erfinder: Habeck, Thorsten, Dr., 67149 Meckenheim (DE); Wünsch, Thomas, Dr., 67346 Speyer (DE); Westenfelder, Horst, 67435 Neustadt (DE)

(56) Entgegenhaltungen:
- WO-A-91/11989
- WO-A-96/15102
- DE-A- 19 755 649
- DATABASE WPI Section Ch, Week 199513 Derwent Publications Ltd., London, GB; Class B05, AN 1995-093802 XP002132962 & JP 07 017912 A (SHISEIDO CO LTD), 20. Januar 1995 (1995-01-20)

## Beschreibung

Die vorliegende Erfindung betrifft neue oligomere 4,4-Diarylbutadiencarbonsäureester und ihre Verwendung als Lichtschutzmittel sowie ein Verfahren zu deren Herstellung.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-Aund UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

US 4,950,467 beschreibt die Verwendung von 2,4-Pentadiensäurederivaten als UV-Absorber in kosmetischen Präparaten. Die in dieser Patentschrift bevorzugt genannten Monoaryl-substituierten Verbindungen haben ebenfalls den Nachteil, daß sie nicht genügend photostabil sind.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstruktur aufweisen und je nach Substituent in Öl oder Wasser löslich sind.

Die Aufgabe wurde in der nicht-vorveröffentlichten DE-A 19755649 (O.Z. 48641) mit monomeren 4,4-Diarylbutadienen, die in 1-Stellung funktionalisiert sind, bereits im wesentlichen gelöst, mit der Einschränkung, daß die Aufgabe fortbestand, Lichtschutzmittel zu finden, die neben den genannten Eigenschaften eine geringe Hauptpenetration aufweisen.

Diese Aufgabe wurde erfindungsgemäß gelöst mit neuen oligomeren 4,4-Diarylbutadiencarbonsäureestern der Formel I 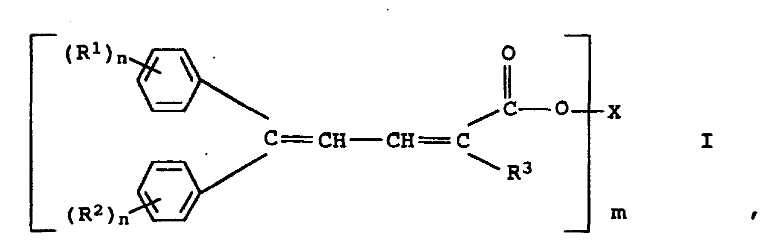 in der
- R¹ und R²: unabhängig voneinander Wasserstoff, C₁-C₁₂-Alkyl, C₁-C₈-Alkoxy, C₁-C₁₂-Alkylamino, C₁-C₁₂-Dialkylamino bedeuten;
- R³: Wasserstoff, COOR⁵, COR⁵, CONR⁵R⁶, CN, C₁-C₁₂-Alkyl, C₃-C₆-Cycloalkyl, C₇-C₁₀-Bicycloalkyl, Phenyl, Naphthyl, wobei Cycloalkyl, Phenyl und Naphthyl gegebenenfalls substituiert sind mit einem oder mehreren Resten aus der Gruppe, bestehend aus Fluor, Chlor, Brom, Cyano, Nitro, Amino, C₁-C₄-Alkylamino, C₁-C₄-Dialkylamino, Hydroxy, C₁-C₄-Alkyl und C₁-C₄-Alkoxy bedeutet;
- R⁵ und R⁶: unabhängig voneinander Wasserstoff, C₁-C₁₂-Alkyl, C₃-C₆-Cycloalkyl, C₇-C₁₀-Bicycloalkyl, Phenyl, Naphthyl, wobei Cycloalkyl, Phenyl und Naphthyl gegebenenfalls substituiert sind mit einem oder mehreren Resten aus der Gruppe, bestehend aus Fluor, Chlor, Brom, Cyano, Nitro, Amino, C₁-C₄-Alkylamino, C₁-C₄-Dialkylamino, Hydroxy, C₁-C₄-Alkyl und C₁-C₄-Alkoxy bedeuten;
- m: die Werte 2 oder 4,
- n: die Werte 1 bis 3 und
- X: -CH₂CH₂- oder den Rest des Pentaerythrits der Formel II 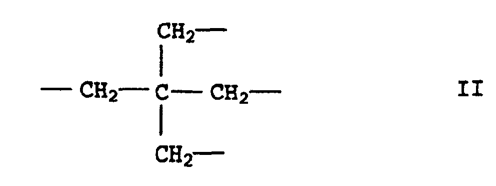
bedeuten,
als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Als Alkylreste R¹ bis R⁶ seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, genannt.

Als Cycloalkylreste seien für R³ bis R⁶ C₃-C₆-Cycloalkylketten, ausgewählt aus der Gruppe, bestehend aus Cyclopropyl, Cyclobutyl, Cyclopentyl, Cyclohexyl, 1-Methylcyclopropyl, 1-Ethylcyclopropyl, 1-Propylcyclopropyl, 1,2-Dimethylcyclopropyl, 1-Methyl-2-Ethylcyclopropyl, genannt.

Die Cyoloalkcnyl und Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Bicycloalkyl reste seien für R³ bis R⁶ gesättigte oder ungesättigte C₇-C₁₀ bicyclische Ringsysteme, insbesondere bicyclische Terpene wie Pinan-, Pinen-, Bornan-, Campherderivate oder auch Adamantan genannt.

Als Alkoxyreste für R¹ und R² kommen solche mit 1 bis 8 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Mono- oder Dialkylaminoreste für R¹ und R² kommen solche in Betracht, die Alkylreste mit 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy substituiert sein können. Bevorzugt sind ggf. substituiertes Phenyl, Methoxyphenyl und Naphthyl.

Als C₁-C₁₂-Alkylreste seien für R¹ bis R⁶ besonders bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 2-Ethylhexyl genannt.

Als Cycloalkylreste seien für R³ bis R⁶ besonders bevorzugt Cyclopentyl und Cyclohexyl genannt.

Als Mono- oder Dialkylaminoreste kommen für R¹ und R² besonders bevorzugt Methyl-, Ethyl-, n-Propyl-, n-Butyl-, 2-Methylpropyl-, 1,1-Dimethylpropyl-, 2-Ethylhexyl in Betracht.

Als Bicycloalkylreste seien für R³ bis R⁶ besondere bevorzugt Campherderivate genannt.

Die Substituenten R¹ und R² können jeweils in ortho, meta und/oder para Position am Aromaten gebunden sein. Im Falle von disubstituierten Aromaten (n = 2) können R¹ und R² in ortho/para oder meta/para Position vorliegen. Bevorzugt sind Verbindungen der Formel I mit n = 1, in denen R¹ gleich R² ist und beide Reste in der para-Position vorliegen.

Ganz besonders bevorzugt sind solche Verbindungen der Formel I, in der
- R¹ und R²: unabhängig voneinander Wasserstoff, C₁-C₁₂-Alkyl, C₁-C₈-Alkoxy,
- R³: Wasserstoff, COOR⁵, COR⁵, CONR⁵R⁶, CN, C₃-C₆-Cycloalkyl oder C₇-C₁₀-Bicycloalkyl bedeuten.

Ebenfalls bevorzugt ist die Verwendung solcher Verbindungen der Formel I, in der
- R¹ und R²: unabhängig voneinander Wasserstoff, C₁-C₁₂-Alkyl, C₁-C₈-Alkoxy,
- R³: COOR⁵, COR⁵, CONR⁵R⁶;
- R⁵ und R⁶: unabhängig voneinander Wasserstoff, C₁-C₁₂-Alkyl, C₃-C₆-Cycloalkyl, C₇-C₁₀-Bicycloalkyl, Phenyl, Naphthyl, gegebenenfalls substituiert und
- n: 1 bis 3
bedeuten, da diese Verbindungen besonders photostabil und gleichzeitig farblos sind.

Besonders bevorzugt sind oligomere 4,4-Diarylbutadien-1-carbonsäureester, die in 1-Stellung noch eine Cyangruppe, eine Alkylcarbonyl- oder eine Carbonsäureestergruppe tragen.

Die Herstellung der neuen Verbindungen der Formel I erfolgt durch Knoevenagel-Kondensation von m mol Aldehyden der Formel III 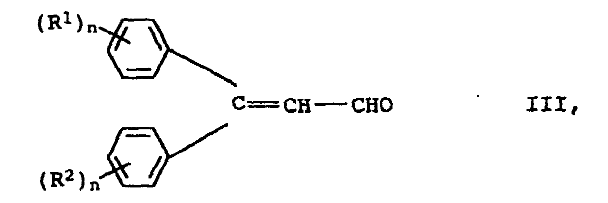 in der R¹, R² und n die oben angegebenen Bedeutungen haben mit oligomeren, gegebenenfalls substituierten Essigsäureestern der Formel IV 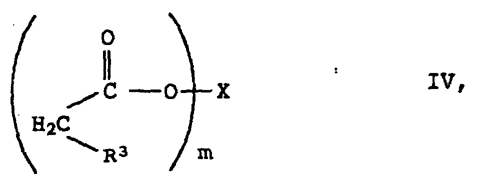 in der R³, X und m die oben angegebenen Bedeutungen haben.

Die Umsetzung kann z.B. in aromatischen Lösungsmitteln wie Toluol oder Xylol durchgeführt werden (s. z.B. Organikum, Ausgabe 1976, S. 572). Bevorzugt werden jedoch polare organische Lösungsmittel wie Dimethylformamid, Dimethylacetamid, N-Methylpyrrolidon, Trialkylorthoformiat oder Alkohole wie n-Propanol, n-Butanol, Ethylenglykol, Diethylenglykol, Ethylenglykolmonomethylether, Cyclohexanol oder ähnliche Verbindungen verwendet. Bilden die verwendeten Ausgangsverbindungen bereits eine flüssige Mischung, kann auf ein zusätzliches Lösungsmittel verzichtet werden. Die Reaktionstemperaturen liegen bevorzugt zwischen 20 und 180°C, besonders bevorzugt zwischen 40 und 150°C. Der Druck ist bevorzugt normaler Atmosphärendruck. In Abhängigkeit von der Reaktivität der eingesetzten Verbindung IV ist die Verwendung eines Katalysators bzw. eines Katalysatorgemisches vorteilhaft. Als Katalysatoren eignen sich z.B. Ammoniumacetat, Piperidin und β-Alanin und deren Acetate.

Als Katalysatoren für die Umsetzung können bei sehr langen Reaktionszeiten zusätzlich Lewis-Säuren wie AlCl₃, ZrCl₄, TiCl₄ oder vor allem ZnCl₂ in den hierfür üblichen Mengen verwendet werden.

Die substituierten Essigester IV können beispielsweise durch Umsetzung von z.B. Cyanessigsäure oder deren Estern mit den entsprechenden Polyolen X(OH)ₘ in Gegenwart eines Katalysators wie Borsäure, Na₂CO₃ oder K₂CO₃ oder Tetrabutylorthotitanat vorzugsweise in Toluol oder Xylol hergestellt werden.

Die erfindungsgemäßen Verbindungen der Formel I können prinzipiell in ihren verschiedenen geometrischen Isomeren, d.h. mit einem Z,Z; Z,E; E,Z und/oder E,E-konfigurierten Diensystem, vorliegen. Bevorzugt als kosmetische Lichtschutzmittel sind die all-E- und/oder all-Z-Isomeren, ganz besonders bevorzugt sind die all-E-Isomeren.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-A- und UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasserin-Öl-Cremes, Öl-in-Wasser-Cremes und -Lotionen, Aerosol-Schaumcremes, Gele, Öle, Fettstifte, Puder, Sprays oder alkoholischwäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminiumund/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium-u. Triethanolaminsalze | 27503-81-7 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |
| 30 | Dimethicon-diethylbenzalmalonat Bis[2-hydroxy-5-tert.-octyl-3- | 207574-74-1 |
| 31 | (benzotriazol-2-yl)phenyl]methan (Bisoctyltriazon) 1H-Benzimidazol-4,6-disulfonsäure, | 103597-45-1 |
| 32 | 2,2'-(1,4-phenylen)bis-, Di-Natriumsalz (Benzimidazylat) Phenol, 2,2'-[6-(4-methoxyphenyl)-1,3,5- | 180898-37-7 |
| 33 | triazin-2,4-diyl]bis[5-[(2-ethylhexyl)oxy] (Aniso Triazin) | 187393-00-6 |

Schließlich sind auch mikronisierte Pigmente wie Titandioxid und Zinkoxid zu nennen.

Zum Schutz menschlicher Haare vor UV-Strahlen können die erfindungsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Verbindungen der Formel I kann auch zur Stabilisierung von Wirk- und Hilfsstoffen in kosmetischen und pharmazeutischen Formulierungen ausgenutzt werden.

Die erfindungsgemäßen Verbindungen eignen sich auch in hervorragender Weise zum Stabilisieren von organischen Materialien gegen die Einwirkung von Licht, Sauerstoff und Wärme.

Als Kunststoffe, die durch die erfindungsgemäßen Verbindungen I stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol sowie Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);
Halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoffe, Polyphenylenether, Polyester, Polycarbonate, Polyoxymethylene, Polysulfone, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen I können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen I zum Stabilisieren von Polyolefinen, insbesondere von Polyethylen, von Polycarbonaten, von Polyamiden, von Polyestern, von Polystyrol, von ABS und von Polyurethanen verwendet. Insbesondere können auch Folien aus den genannten Kunststoffen stabilisiert werden.

Für diese Anwendungsbereiche werden die Verbindungen in Konzentrationen von 0,01 bis 5 Gew.-%, bezogen auf die Menge des Kunststoffs, eingesetzt, bevorzugt in einer Konzentration von 0,02 bis 2 Gew.-%. Die Kombination mit anderen Stabilisatoren, beispielsweise Antioxidantien, Metalldesaktivatoren oder anderen Lichtschutzmitteln sowie mit antistatischen oder flammhemmenden Mitteln, ist oft vorteilhaft. Besonders wichtige Costabilisatoren sind beispielsweise sterisch gehinderte Phenole sowie Phosphite, Phosphonite, Amine und Schwefelverbindungen.

Als geeignete Costabilisatoren kommen z.B. in Betracht:
Phenolische Antioxidationsmittel wie
   2,6-Di-tert.-butyl-4-methylphenol,
   n-Octadecyl-β-(3,5-di-tert.-butyl-4-hydroxyphenol)-propionat,
   1,1,3-Tris-(2-methyl-4-hydroxy-5-tert.-butylphenyl)-butan,
   1,3,5-Trimethyl-2,4,6-tris-(3,5-di-tert.-butyl-4-hydroxybenzyl)-benzol,
   1,3,5-Tris-(3,5-di-tert.-butyl-4-hydroxybenzyl)-isocyanurat,
   1,3,5-Tris-[β-(3,5-di-tert.-butyl-4-hydroxyphenyl)-propionylethyl]-isocyanurat,
   1,3,5-Tris-(2,6-di-methyl-3-hydroxy-4-tert.-butylbenzyl)-isocyanurat und
   Pentaerythrit-tetrakis-[β-(3,5-di-tert.-butyl-4-hydroxy)-propionat],
phosphorhaltige Antioxidantien wie
   Tris-(nonylphenyl)-phosphit, Distearylpentaerythritphosphit,
   Tris-(2,4-di-tert.-butyl-phenyl)-phosphit,
   Tris-(2-tert.-butyl-4-methylphenyl)-phosphit,
   Bis-(2,4-di-tert.-butylphenyl)-pentaerythritdiphosphit und
   Tetrakis-(2,4-di-tert.-butylphenyl)-4,4'-biphenylendiphosphit,
schwefelhaltige Antioxidantien wie
   Dilaurylthiodipropionat,
   Dimyristylthiodipropionat,
   Distearylthiodipropionat,
   Pentaerythrittetrakis-(β-laurylthiopropionat) und
   Pentaerythrittetrakis-(β-hexylthiopropionat),
sterisch gehinderte Amine wie
   Bis-(2,2,6,6-tetramethylpiperidyl)-sebacat,
   Bis-(1,2,2,6,6-pentamethylpiperidyl)-sebacat,
   Bis-(1,2,2,6,6-pentamethylpiperidyl)-ester,
   N,N'-Bis(formyl)-bis(2,2,6,6-tetramethyl-4-piperidyl)-1,6-hexandiamin,
   das Kondensationsprodukt von
   1-Hydroxy-2,2,6,6-tetramethyl-4-hydroxypiperidin und Bernsteinsäure,
   das Kondensationsprodukt von
   N,N'-(2,2,6,6-Tetramethylpiperidyl)-hexamethylendiamin und
   4-tert.-Octylamino-2,6-dichlor,1,3,5-s-triazin,
   Poly-[3-(Eicosyl/Tetracosyl)-1-(2,2,6,6-tetramethylpiperidin-4-yl)-pyrrolidin-2,5-dion],
   Tris-(2,2,6,6-Tetramethylpiperidyl)-nitrilotriacetat,
   Tetrakis-(2,2,6,6-tetramethyl-4-piperidyl)-1,2,3,4-butantetracarbonsäure,
   1,1'-(1,2-Ethandiyl)-bis-(3,3,5,5-tetramethylpiperazinon),
   die Kondensationsprodukte von
   4-Amino-2,2,6,6-tetramethylpiperidinen und Tetramethylolacetylendiharnstoffen sowie
   2-(2'-Hydroxyphenyl)-benztriazole,
   2-Hydroxybenzophenone,
   Arylester von Hydroxybenzoesäuren,
   α-Cyanozimtsäurederivate,
   Nickelverbindungen oder
   Oxalsäuredianilide.

Zur Vermischung der erfindungsgemäßen Verbindungen I, vor allem mit Kunststoffen, können alle bekannten Vorrichtungen und Methoden zum Einmischen von Stabilisierungsmitteln oder anderen Zusätzen in Polymere angewandt werden.

Die erfindungsgemäßen oligomeren 4,4-Diarylbutadiencarbonsäureester zeichnen sich durch eine gute Verträglichkeit mit den üblichen Kunststoffarten und durch eine gute Löslichkeit und eine ausgezeichnete Verträglichkeit in den üblichen Lacksystemen aus. Sie haben in der Regel keine oder nur eine sehr geringe Eigenfarbe, sind bei den üblichen Kunststoff- und Lack-Verarbeitungstemperaturen stabil und nicht flüchtig und bewirken eine lange Schutzdauer der mit ihnen behandelten Materialien.
Vor allem jedoch zeigen sie praktisch keine Migrationsneigung in Kunststoffen.

### Beispiele

### Beispiel 1

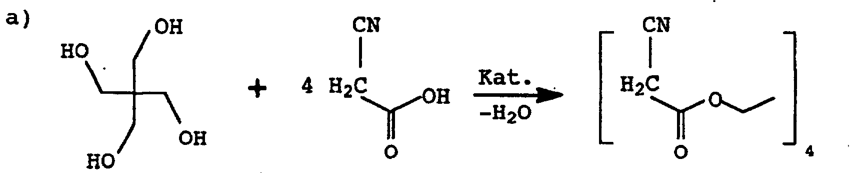

0,1 mol Cyanessigsäure wurden mit 0,025 mol Pentaerythrit in 200 ml Xylol suspendiert. Man setzte 2 g para-Toluolsulfonsäure hinzu und erhitzte unter Rückflußkühlung zum Sieden, wobei das entstehende Reaktionswasser am Wasserabscheider entfernt wurde. Nach Abkühlen auf Raumtemperatur filtrierte man vom Produkt ab, wusch zweimal mit Wasser nach und trocknete bei 50°C im Vakuum (200 mbar). Ausbeute 80 % d. Theorie. 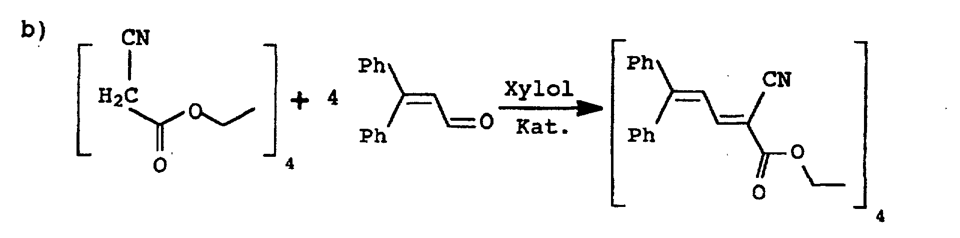 0,05 mol der Verbindung aus 1 a) wurden mit 0,2 mol β-Phenylzimtaldehyd in 150 ml Xylol suspendiert. Nach Zugabe von 2 ml Piperidin und 2 ml Eisessig wurde unter Rückflußkühlung zum Sieden erhitzt. Die Reaktion war nach ca. 6 h beendet (DC-Kontrolle). Man saugte ab und kristallisierte aus Xylol um.
Ausbeute 70 % d. Theorie.
λ_{max.} : 352; E¹₁ : 810

### Beispiel 2

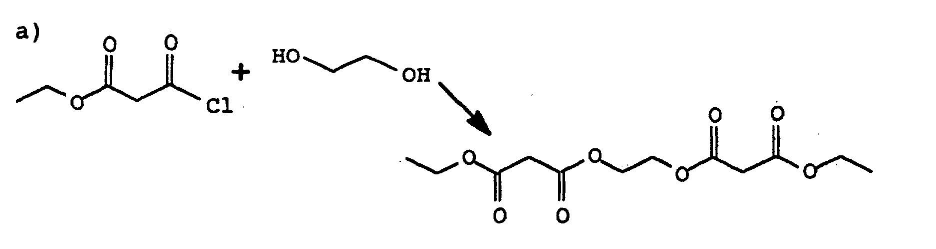

0,0475 mol Ethylenglykol und 0,1 mol Triethylamin wurden in 100 ml Toluol gelöst, die Lösung bei 15 bis 20°C vorsichtig mit 0,1 mol Malonsäurechloridmonoethylester versetzt und 2 h bei Raumtemperatur nachgerührt. Man setzte 200 ml Wasser hinzu und trennte die Phasen. Die organische Phase wurde zweimal mit 150 ml Wasser und einmal mit gesättigter NaHCO₃-Lösung gewaschen. Nach Trocknen über Na₂SO₄ engte man ein und erhielt ein schwach gelbes Öl.
Ausbeute 58 % d. Theorie. 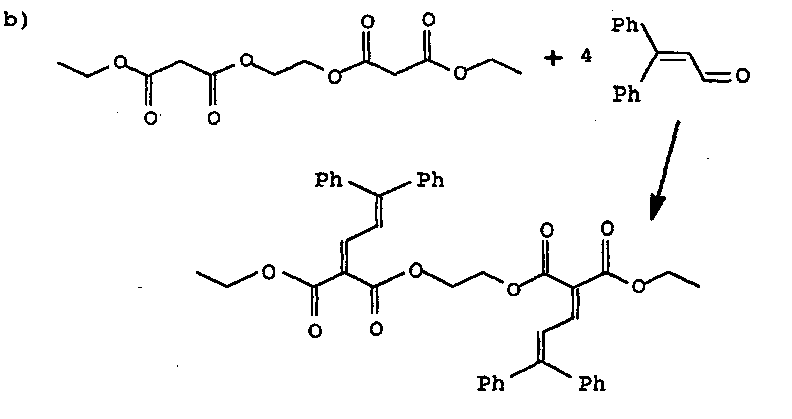 0,025 mol der Verbindung aus 2 a) und 0,05 mol β-Phenylzimtaldehyd wurden in 100 ml Ethanol gelöst und mit 2 ml Piperidin und 2 ml Eisessig versetzt. Man erhitzte 5 h unter Rückflußkühlung zum Sieden, setzte Wasser zu und trennte das sich abscheidende Öl ab. Die wäßrige Phase wurde mit Methylenchlorid extrahiert und die vereinigten organischen Phasen wurden über Na₂SO₄ getrocknet und eingeengt.
Ausbeute 60 % d. Theorie gelbes zähflüssiges Öl
λₘₐₓ. : 336; E¹₁ : 800

### Beispiel 3

In gleicher Weise wie in Beispiel 1 beschrieben wurde die Verbindung der Formel 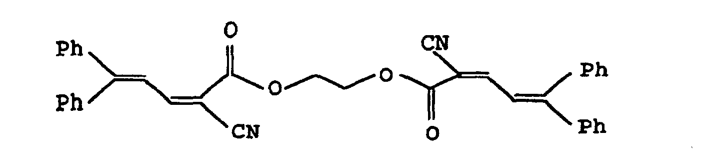 λ_{max.} : 354; E¹₁ : 850
hergestellt.

### Beispiel 4

### (Kosmetische Zubereitung)

Sonnencreme (LSF 20)
Massengehalt
(Gew.-%)
- ad 100: Wasser
- 8,00: Octyl Methoxycinnamat
- 8,00: Titanium Dioxid
- 6,00: PEG-7-Hydrogenated Castor Öl
- 5,00: Verbindung des Beispiels 1
- 6,00: Mineralöl
- 5,00: Zink Oxid
- 5,00: Isopropyl Palmitat
- 5,00: Imidazolidinyl Urea
- 3,00: Jojobaöl
- 2,00: PEG-45/Dodecyl Glycol Copolymer
- 1,00: 4-Methylbenzyliden Campher
- 0,60: Magnesium Stearat
- 0,50: Tocopheryl Acetat
- 0,25: Methylparaben
- 0,20: Disodium EDTA
- 0,15: Propylparaben

## Patentansprüche

1. Oligomere 4,4-Diarylbutadiencarbonsäureester der Formel I 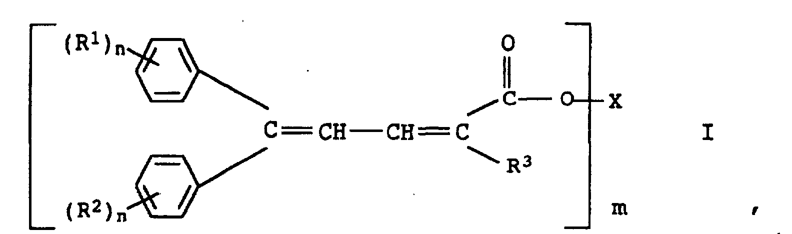 in der
R¹ und R² unabhängig voneinander Wasserstoff, C₁-C₁₂-Alkyl, C₁-C₈-Alkoxy, C₁-C₁₂-Alkylamino, C₁-C₁₂-Dialkylamino bedeuten;
R³ Wasserstoff, COOR⁵, COR⁵, CONR⁵R⁶, CN, C₁-C₁₂-Alkyl, C₃-C₆-Cycloalkyl, C₇-C₁₀-Bicycloalkyl, Phenyl, Naphthyl, wobei Cycloalkyl, Phenyl und Naphthyl gegebenenfalls substituert sind mit einem oder mehreren Resten aus der Gruppe, bestehend aus Fluor, Chlor, Brom, Cyano, Nitro, Amino, C₁-C₄-Alkylamino, C₁-C₄-Dialkylamino, Hydroxy, C₁-C₄-Alkyl und C₁-C₄-Alkoxy bedeutet;
R⁵ und R⁶ unabhängig voneinander Wasserstoff, C₁-C₁₂-Alkyl, C₃-C₆-Cycloalkyl, C₇-C₁₀-Bicycloalkyl, Phenyl, Naphthyl, wobei Cycloalkyl, Phenyl und Naphthyl gegebenenfalls substituert sind mit einem oder mehreren Resten aus der Gruppe, bestehend aus Fluor, Chlor, Brom, Cyano, Nitro, Amino, C₁-C₄-Alkylamino, C₁-C₄-Dialkylamino, Hydroxy, C₁-C₄-Alkyl und C₁-C₄-Alkoxy bedeuten;
m die Werte 2 oder 4,
n die Werte 1 bis 3 und
X -CH₂CH₂- oder den Rest des Pentaerythrits der Formel II 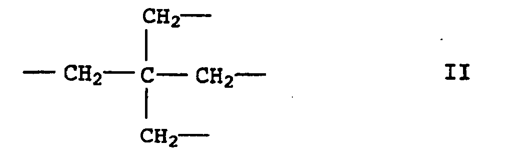
bedeuten.

2. Verwendung von oligomeren 4,4-Diarylbutadiencarbonsäureestern der Formel I gemäß Anspruch 1 als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

3. Verwendung von Verbindungen der Formel I gemäß Anspruch 2 als photostabile UV-A-Filter.

4. Lichtschutzmittel enthaltende kosmetische und pharmazeutische Zubereitungen zum Schutz der menschlichen Epidermis oder der menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische Zubereitungen bekannten im UV-Bereich absorbierenden Verbindungen, Verbindungen der Formel I gemäß Anspruch 1 enthalten.

5. Verfahren zur Herstellung von Verbindungen der Formel I gemäß Anspruch 1, **dadurch gekennzeichnet, daß** man oligomere, gegebenenfalls substituierte Essigsäureester der Formel IV 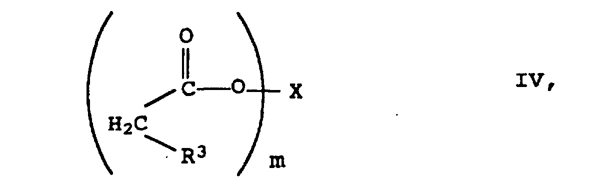 mit m mol von Verbindungen der Formel III 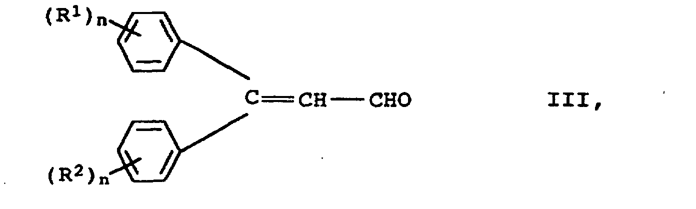 umsetzt,
in denen R¹, R², R³, X, m und n die in Anspruch 1 angegebenen Bedeutungen haben.

## Claims

1. An oligomeric 4,4-diarylbutadienecarboxylate or -carboxamide of the formula I 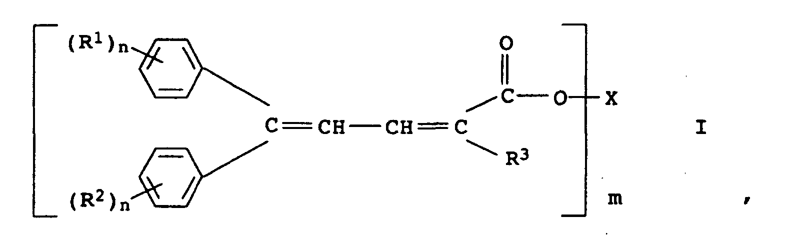 in which
R¹ and R², independently of one another, are hydrogen, C₁-C₁₂-alkyl, C₁-C₈-alkoxy, C₁-C₁₂-alkylamino, C₁-C₁₂-dialkylamino;
R³ is hydrogen, COOR⁵, COR⁵; CONR⁵R⁶, CN, C₁-C₁₂-alkyl, C₃-C₆-cycloalkyl, C₇-C₁₀-bicycloalkyl, phenyl, naphthyl, where cycloalkyl, phenyl and naphthyl are optionally substituted by one or more radicals from the group consisting of fluorine, chlorine, bromine, cyano, nitro, amino, C₁-C₄-alkylamino, C₁-C₄-dialkylamino, hydroxyl, C₁-C₄-alkyl and C₁-C₄-alkoxy;
R⁵ and R⁶, independently of one another, are hydrogen, C₁-C₁₂-alkyl, C₃-C₆-cycloalkyl, C₇-C₁₀-bicycloalkyl, phenyl, naphthyl, where cycloalkyl, phenyl and naphthyl are optionally substituted by one or more radicals from the group consisting of fluorine, chlorine, bromine, cyano, nitro, amino, C₁-C₄-alkylamino, C₁-C₄-dialkylamino, hydroxyl, C₁-C₄-alkyl and C₁-C₄-alkoxy;
m is 2 or 4,
n is from 1 to 3, and
X is -CH₂CH₂- or the pentaerythritol radical of the formula II

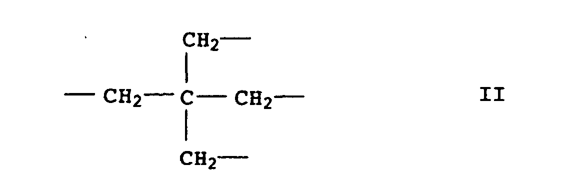

2. The use of an oligomeric 4,4-diarylbutadienecarboxylate of the formula I as claimed in claim 1 as a photostable UV filter in cosmetic and pharmaceutical preparations for protecting the human skin or human hair against solar rays, alone or together with compounds which absorb in the UV region and are known per se for cosmetic and pharmaceutical preparations.

3. The use of a compound of the formula I as claimed in claim 2 as a photostable UV-A filter.

4. A cosmetic or pharmaceutical preparation comprising a sunscreen for protecting the human epidermis or human hair against UV light in the range from 280 to 400 nm, which comprises, in a cosmetically and pharmaceutically suitable carrier, alone or together with compounds which absorb in the UV region and which are known per se for cosmetic and pharmaceutical preparations, compounds of the formula I as claimed in claim 1.

5. A process for the preparation of compounds of the formula I as claimed in claim 1, which comprises reacting oligomeric, optionally substituted acetic esters of the formula IV 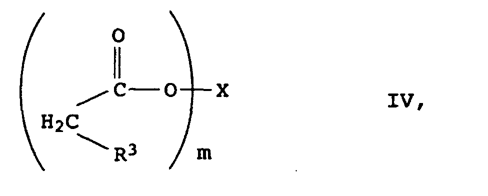 with m mol of compounds of the formula III 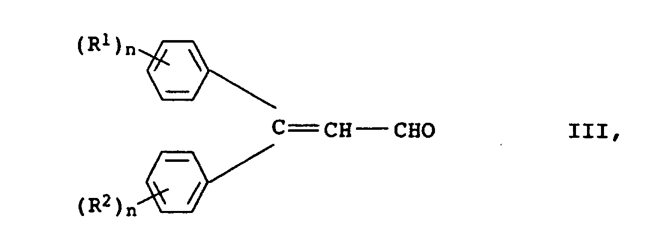 in which R¹, R², R³, X, m and n are as defined in claim 1.

## Revendications

1. Esters d'acide 4,4-diarylbutadiènecarboxylique oligomères de formule I 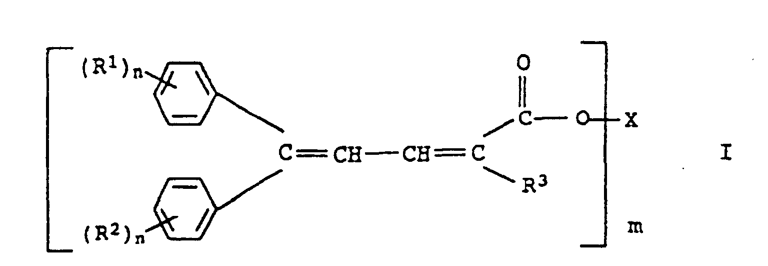 dans laquelle
R¹ et R² désignent, indépendamment l'un de l'autre, un reste hydrogène, alkyle en C₁-C₁₂, alcoxy en C₁-C₈, alkyl(C₁-C₁₂)amino, dialkyl(Ci-C12)amino;
R³ représente un reste hydrogène, COOR⁵, COR⁵, CONR⁵R⁶, CN, alkyle en C₁-C₁₂, cycloalkyle en C₃-C₆, bicycloalkyle en C₇-C₁₀, phényle, naphtyle, tandis que les groupes cycloalkyle, phényle et naphtyle sont éventuellement substitués par un ou plusieurs restes choisis dans le groupe consistant en fluor, chlore, brome, cyano, nitro, amino, alkyl(C₁-C₄)amino, dialkyl(C₁-C₄)amino, hydroxy, alkyle en C₁-C₄ et alcoxy en C₁-C₄;
R⁵ et R⁶ désignent, indépendamment l'un de l'autre, un reste hydrogène, alkyle en C₁-C₁₂, cycloalkyle en C₃-C₆, bicycloalkyle en C₇-C₁₀, phényle, naphtyle, tandis que les groupes cycloalkyle, phényle et naphtyle sont éventuellement substitués par un ou plusieurs restes choisis dans le groupe consistant en fluor, chlore, brome, cyano, nitro, amino, alkyl(C₁-C₄)amino, dialkyl(C₁-C₄)amino, hydroxy, alkyle en C₁-C₄ et alcoxy en C₁-C₄;
m a les valeurs 2 ou 4;
n possède les valeurs 1 à 3 et
X représente -CH₂CH₂- ou le reste de pentaérythrite de formule II

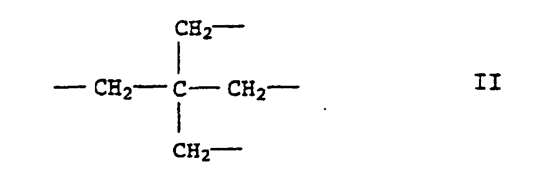

2. Utilisation d'esters d'acide 4,4-diarylbutadiènecarboxylique oligomères de formule I selon la revendication 1 comme filtre UV photostable dans des compositions cosmétiques et pharmaceutiques pour la protection de la peau humaine ou des cheveux humains contre les rayons solaires, seuls ou conjointement avec des composés absorbant dans le domaine UV, connus pour des compositions cosmétiques et pharmaceutiques.

3. Utilisation de composés de formule I selon la revendication 2 comme filtre UV-A photostable.

4. Compositions cosmétiques et pharmaceutiques contenant des agents de protection contre la lumière, pour la protection de l'épiderme humain ou des cheveux humains contre la lumière UV dans le domaine de 280 à 400 nm, **caractérisé par le fait qu'**elles contiennent dans un support approprié en cosmétique ou en pharmacie, seuls ou conjointement avec des composés absorbant dans le domaine UV, connus en soi pour des compositions cosmétiques et pharmaceutiques, des composés de formule I selon la revendication 1.

5. Procédé pour la préparation de composés de formule I selon la revendication 1, **caractérisé par le fait qu'**on fait réagir des esters d'acide acétique oligomères, éventuellement substitués, de formule IV 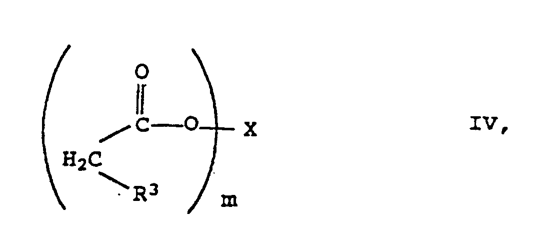 avec m mol de composés de formule III 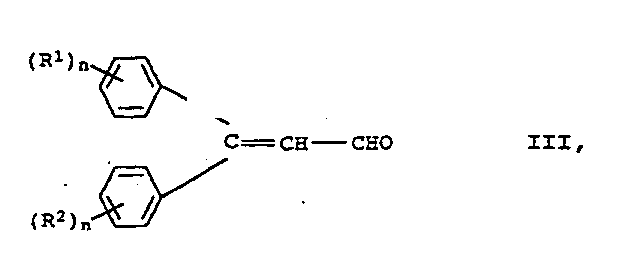 dans lesquels R¹, R², R³, X, m et n ont les significations indiquées dans la revendication 1.
